(19) Europäisches Patentamt
European Patent Office
Office européen des brevets

(11) Publication number : 0 436 960 B1

# (12) EUROPEAN PATENT SPECIFICATION

(45) Date of publication of patent specification : 08.09.93 Bulletin 93/36

(51) Int. Cl.$^5$ : **A61K 7/00**, C08F 220/04

(21) Application number : **90125800.4**

(22) Date of filing : **29.12.90**

## (54) Cosmetic or pharmaceutical formulations comprising crosslinked carboxylic copolymers useful as thickeners.

(30) Priority : **11.01.90 US 463433**

(43) Date of publication of application : **17.07.91 Bulletin 91/29**

(45) Publication of the grant of the patent : **08.09.93 Bulletin 93/36**

(84) Designated Contracting States : **DE ES FR GB IT NL**

(56) References cited :
**DE-C- 2 949 843**
**US-A- 4 360 651**
**US-A- 4 800 220**

(73) Proprietor : **RHEOX INTERNATIONAL, INC.**
**Wyckoffs Mill Road**
**Hightstown, New Jersey 08520 (US)**

(72) Inventor : **Santhanam, Mahalingam**
**12 Covington Drive**
**East Windsor, NJ 08520 (US)**
Inventor : **Reichert, William**
**17 Willow Brook Road**
**Freehold, NJ 07798 (US)**

(74) Representative : **Strehl Schübel-Hopf Groening & Partner**
**Maximilianstrasse 54**
**D-80538 München (DE)**

EP 0 436 960 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

### Background Of The Invention

The present invention relates to a cosmetic or pharmaceutical formulation compris crosslinked carboxylic copolymers useful as thickeners.

### Discussion Of The Prior Art

Materials that are capable of thickening aqueous compositions find a variety of applications in cosmetic and pharmaceutical formulations, as well as other personal care products, in the form of creams, pastes, gels, ointments, and emulsions. Such materials are also useful in textile printing inks, drilling muds, and aqueous coating compositions. Numerous thickeners are currently employed throughout the industry, such as natural gums, for example, acacia, aliginate, cariageenan, guar, karaya, pectin, tragacanth, and xanthane; cellulose derivatives such as carboxymethyl cellulose, hydroxyethyl cellulose, and methyl hydroxypropyl cellulose; inorganic materials such as clays and fumed silica; and synthetic polymers such as acrylic acid based copolymers and polyoxypropylene/ethylene block polymers.

All of the prior art materials exhibit specific benefits and associated disadvantages for different aqueous systems. For example, two cellulose-derived substances that are widely employed throughout the industry are sodium carboxymethyl cellulose and nonionic hydroxyethyl cellulose. Both materials provide high thickening efficiency in water at low concentrations. However, these materials, and natural gums in general, are subject to biodegradation. Furthermore, although the cellulose derivatives provide excellent performance in terms of thickening, they do not impart to personal products in particular such features as superior feel and appearance that boost the consumer appeal for such products.

The use of synthetic polymers as thickeners has become widespread throughout the personal care product industry due to their resistance to biodegradation and their ability to thicken aqueous compositions with a smooth feel, smooth texture, and in certain instances, provide clear gels. The most commonly employed synthetic polymers are lightly crosslinked carboxylic polymers prepared from unsaturated carboxylic acid containing monomers such as acrylic acid, methacrylic acid, and maleic anhydride, and are described in U.S. Patent No. 2,798,053. The high thickening efficiency of these polymers is achieved by crosslinking acrylic acid based polymers with a polyalkenylpolyether of a polyhydric alcohol. The crosslinking renders the polymers water-swellable or water-dispersible.

Other known carboxylic polymers which maintain viscosities in aqueous solutions containing inorganic ions or salts are described in U.S. Patent Nos. 3,915,921; 3,940,351; and 4,509,949. The copolymers consist of an unsaturated carboxylic acid monomer and one or more alkyl acrylate esters wherein the alkyl groups contain 10 to 30 carbon atoms and, optionally, a crosslinking monomer which is a polyfunctional vinylidene monomer containing at least two polymerizable $CH_2$ = C< groupings, the unsaturated bonds of the polymerizable groupings being non-conjugated with respect to each other.

Processes for preparing the above described carboxylic polymers in a non-toxic, moderately polar solvent are described in U.S. Patent No. 4,267,103. Processes for preparing the carboxylic polymers with a low residual monomer content in a moderately polar solvent are described in U.S. Patent No. 4,758,641.

U.S. Patent No. 4,800,220 discloses crosslinked carboxylic copolymers for providing better resistance to viscosity losses in the presence of dissolved salts. Such copolymers are obtained by copolymerization of an unsaturated carboxylic monomer, a crosslinking agent, an acrylic or methacrylic ester with a polyalkylene glycol, and optionally, an alkyl acrylate or methacrylate.

U.S. Patent No. 4,167,502 discloses polymers of a polymerizable mixture comprising an ethylenically unsaturated acid and an oligomer of an alkyl containing ester of acrylic acid or methacrylic acid which are useful as thickening and bodying agents when partially or completely neutralized in cosmetics, personal care products, latex paints, and oil well drilling compositions.

U.S. Patent Nos. 4,138,381 and 4,230,844 disclose polymeric thickeners and processes for preparing the thickeners which provide both good flow and leveling properties, as well as sag resistance to aqueous coating compositions. The thickeners are prepared by conducting polymerization in a glycol such as ethylene glycol or propylene glycol with or without a little added water.

U.S. Patent No. 4,062,817 discloses carboxylic polymers comprising an unsaturated carboxylic acid, at least one alkyl acrylate or methacrylate ester in which the alkyl group contains 10 to 30 carbon atoms, and a second acrylate or methacrylate ester containing an alkyl group of from 1 to 9 carbon atoms, and optionally, a crosslinking monomer which is a polyfunctional vinylidene monomer containing at least two polymerizable $CH_2$ = C< groupings which rapidly absorb and retain large quantities of water and ionic fluids and are useful

in disposable nonwoven articles.

U.S. Patent Nos. 4,668,731, 4,686,254, 4,778,737, 4,362,715, 3,953,591, and Canadian Patent No. 1,067,411 describe the utilization of carboxylic copolymers as thickeners. The use of ethoxylated surface-active agents in combination with carboxyl polymers is also well known in the art. U.S. Patent Nos. 4,375,533, 4,419,502, and 4,526,937 disclose the use of ethoxylated surface-active agents during the polymerization of crosslinked carboxylic copolymers to facilitate the polymerization process, specifically, to reduce polymer build-up within the reactor, control the particle size of the precipitated polymer, and attain a higher solids content during polymerization.

U.S. Patent No. 4,420,596 discloses a method for polymerizing olefinically unsaturated carboxylic acids in mineral spirits in the presence of a free radical forming catalyst and at least two surface active agents having HLB values of less than 10, a sorbitan ester with a glycerol or alkylene glycol ester and a long chain alcohol, to provide fluid, high total-solid dispersions of polymer in mineral spirits.

U.S. Patent No. 4,536,528 discloses the addition of a linear or branched copolymer of propylene oxide and ethylene oxide as well as glyceryl tri-12-hydroxystearate and/or mixed saturated $C_{18}$-$C_{36}$ fatty acid triglycerides to dispersions or slurries of carboxyl-containing polymers in mineral spirits to reduce settling.

U.S. Patent Nos. 4,360,651 and 4,435,524 disclose processes for emulsion polymerization wherein crosslinked acrylic acid copolymers are used in combination with surfactants as dispersants or colloid stabilizers.

U.S. Patent Nos. 4,613,483, 4,696,983, and 4,724,126 disclose methods for eliminating or substantially reducing undesirable polymer build-up on reactor surfaces during the polymerization of vinylidene monomers wherein carboxyl containing polymers and surfactant combinations are deposited on the polymerization reaction vessel walls.

DE-C-29 49 843 discloses a process for preparing carboxylic acid polymers in the presence of a protective colloid which is defined as polyvinylpyrrolidone with a water content of at most 1% by weight. The polymers prepared in this process are highly effective thickeners which can be used in the field of pharmaceuticals, cosmetics, paper, textiles, adhesives and dispersion caotings. The polymers of this reference consist of the following monomers:

- an unsaturated carboxylic acid, such as acrylic acid,
- an additional monoolefinic unsaturated compound, such as vinyl acetate, and
- a crosslinking agent having at least two ethylenically unsaturated double bounds.

Triallylcyanurate is cited as a suitable crosslinking agent; however, when the polymers are to be used as thickeners in aqueous systems, crosslinking agents which do not tend to cleave when exposed to acid or basic conditions are preferred.

Furthermore comparative example 1 shows that when the polymers are prepared in absence of the protective colloid a highly viscous suspension is obtained which is not suitable as a thickener.

The carboxylic polymers described in the prior art are useful as thickeners for aqueous media and, in some cases, hydroalcoholic solvents. The maximum thickening ability of these polymers is attained upon neutralization of the carboxylic acid groups with a base such as ammonium hydroxide, sodium hydroxide, potassium hydroxide, or an amine. However, particularly in the field of cosmetic formulations, new thickeners are desired that are more efficient and provide better emulsion stability properties in combination with better feel and rub-up qualities than prior art thickeners.

## Summary Of The Invention

The present invention overcomes the problems and disadvantages of the prior art by using crosslinked carboxylic copolymers that are useful as thickeners in aqueous systems and achieve superior emulsion stability, non-tackiness and rub-up qualities than prior art thickeners.

It is an object of the invention to provide a cosmetic or pharmaceutical formulation comprising an improved thickener, i.e., a rheology modifier, suspension additive or emulsifying agent, for aqueous and hydroalcoholic systems, having superior emulsion stability, superior feel and appearance, when in form of aqueous formulations such as creams, lotions and gels

Additional objects and advantages of the invention will be set forth in part in the description which follows, and in part will be obvious from the description, or may be learned by practice of the invention. The objects and advantages of the invention will be realized and attained by means of the instrumentalities and combinations, particularly pointed out in the appended claims.

To achieve the objects and in accordance with the purpose of the invention, as embodied and broadly described herein, the invention provides a cosmetic or pharmaceutical formulation for application to the skin, in the form of a thickened emulsion comprising crosslinked carboxylic copolymers formed from a mixture of monomers comprising (a) an monoolefinically unsaturated carboxylic acid; (b) a crosslinking monomer selected from

the group consisting of triallylisocyanurate, triallyl trimellitate and glyoxal bis(diallyl acetal). The carboxylic copolymers used in the invention may further comprise (c) an additional comonomer containing a polymerizable ethylenically unsaturated group; and/or (d) an ethoxylated glyceride compound of the formula

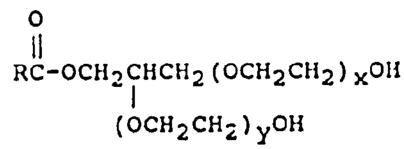

(I)

wherein R represents an alkyl group of from $C_8$ to $C_{18}$ and the sum of x + y is from 20 to 300. The additional comonomer containing a polymerizable ethylenically unsaturated group is not selected from the group consisting or alkyl acrylates, alkyl methacrylates, acrylic acid esters derived from a polyalkylene glycol and methacrylic acid esters derived from a polyalkylene glycol, since the carboxylic copolymers of the invention are not intended for use in salt or ionic environments and the presence of the acrylic acid and methacrylic acid esters do not add to the performance of the carboxylic copolymers of the invention.

The carboxylic copolymers used in the invention are alkali swellable and lightly crosslinked and are useful as thickeners and stabilizers, particularly for cosmetic formulations, including gels, creams and lotions, for personnel care products, and pharmaceuticals.

The inventive formulation comprising carboxylic copolymers containing a crosslinking monomer selected from the group consisting of triallylisocyanurate, triallyl trimellitate, and (glyoxal bis(diallyl acetal), have superior feel and rub-up qualities and excellent emulsion stability to aqueous and hydroalcoholic cosmetic formulations compared to formulation comprising prior art thickeners. Gels, lotions and creams have a non-shingy and non-tacky feel; furthermore the use of multiple stabilizing, suspending and thickening additives is not required.

The carboxylic copolymers used in the invention contain crosslinking monomers which are not commonly utilized as crosslinking agents for water-swellable thickeners, and in particular, have not been used as crosslinking agents for acrylic copolymers which thicken non salt-containing media, but are typically employed or the crosslinking of synthetic rubbers and plastics to achieve improved mechanical properties. In this regard, the art has not recognized crosslinked carboxylic copolymers which comprise this specific class of crosslinking agents and an unsaturated carboxylic acid for thickening aqueous systems in the absence of a salt environment.

The inventors have also discovered that the addition of an ethoxylated glyceride surfactant ester with an HLB value greater than 10 to the carboxylic copolymers used in the invention provide thickeners that have superior non-tackiness, feel and rub-up characteristics than prior art thickeners. The ethoxylated glyceride employed in the present invention is prepared by the ethoxylation of an acyl glyceride, e.g., ethoxylation of a monoglyceride. The surfactant ester, which has a high affinity for the polymer, is added to a non-hydrogen bonding solvent for the polymerization reaction in which it is not soluble. The monomers which comprise the polymer are soluble in the polymerization solvent. As the polymer is formed, the polymer precipitates and the surfactant ester is adsorbed on the surface of the polymer.

Further, aqueous gels containing the crosslinked carboxylic copolymers of the invention, which also contain an ethoxylated glyceride, have improved clarity.

## Description Of The Preferred Embodiments

Reference will now be made in detail to the present preferred embodiments of the invention.

The carboxylic copolymers of the invention may be prepared by copolymerizing (a) from about 70% to about 99%, more preferably from about 92% to about 95% by weight of at least one unsaturated carboxylic monomer; (b) from about 0.8% to about 1.2%, more preferably from about 0.9% to about 1.1% by weight of at least one crosslinking monomer selected from the group consisting of triallylisocyanurate, triallyl trimellitate and glyoxal bis(diallyl acetal); and optionally (c) from about 2% to about 15% by weight of a monomer containing a polymerizable ethylenically unsaturated group, wherein said group is not selected from the group consisting of alkyl acrylates, alkyl methacrylates, acrylic acid esters derived from a polyalkylene glycol and methacrylic acid esters derived from a polyalkylene glycol; and/or (d) from about 0% to about 1.0%, more preferably from about 0.5% to about 0.75% by weight of an ethoxylated glyceride of the formula

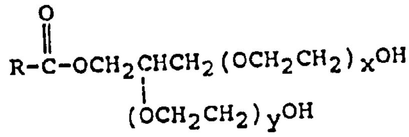

(I)

wherein R represents an alkyl group of from $C_8$ to $C_{18}$ and the sum of x + y is from 20 to 300.

Most preferably, the carboxylic copolymers used in the invention are prepared by copolymerizing (a) about 92% by weight of at least one unsaturated carboxylic monomer; (b) about 1% by weight of at least one crosslinking agent selected from the group consisting of triallyisocyanurate, triallyl trimellitate and glyoxal bis(diallyl acetal); (c) from about 6% to about 7% by weight of a monomer containing a polymerizable ethylenically unsaturated group and optionally (d) about 0.5% by weight of an ethoxylated glyceride of the formula (I).

The unsaturated carboxylic monomers employed in the formulation of the invention are preferably olefinically unsaturated carboxylic acids containing at least one activated carbon-carbon double bond and at least one carboxylic acid group wherein the olefinic double bond is present in the carboxylic acid monomer in either an alpha, beta position relative to the carboxyl group or in the form of a methylene end group such as $CH_2$ = C<. Such exemplary suitable olefinically unsaturated carboxylic acids include acrylic acid, methacrylic acid, ethacrylic acid, fumaric acid, crotonic acid, itaconic acid, styrylacrylic acid, glutaconic acid, maleic acid and acid anhydrides such as maleic anhydride. More preferably, acrylic acid or methacrylic acid are employed; most preferably acrylic acid is employed.

Most preferred crosslinking monomer for use in the invention is triallylisocyanurate.

Monomers which contain a polymerizable ethylenically unsaturated group are also employed in the copolymers used in the invention, providing that the monomers do not adversely effect the thickening properties of the crosslinked carboxylic monomers. Exemplary suitable copolymerizable monomers contain at least one terminal $CH_2$ = C< group and include vinyl acetate, vinyl pyrrolidinone, methyl vinyl ether, ethyl vinyl ether, acrylamide, methacrylamide, and methyl vinyl ketone.

Alkoxylated glycerides suitable for use in the present invention include ethoxylated glycerides derived from the ethoxylation of a monoglyceride (for example, PEG-20 glyceryl oleate is obtained by the ethoxylation of glyceryl [mono] oleate which contains two -OH groups, each of which may react with ethylene oxide until the total level of PEG equals 20). Exemplary suitable ethoxylated glycerides for use in the invention include those represented by the following structure:

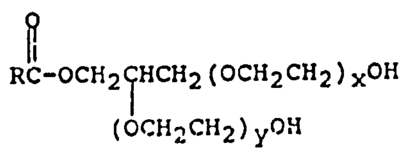

(I)

wherein R represents an alkyl group of from $C_8$ to $C_{18}$ and x + y is from 20 to 300. Exemplary suitable ethoxylated glycerides include PEG-30 glyceryl monococoate (commercially available as Varonic LI-63 from Sherex Chemical Company, Inc.), PEG-78 glyceryl monococoate (commercially available as Varonic LI-67 from Sherex Chemical Company, Inc.), PEG 82 glyceryl monotallowate (commercially available as Varonic LI-48 from Sherex Chemical Company, Inc.), PEG 200 glyceryl monotallowate (commercially available as Varonic LI-420 from Sherex Chemical Company, Inc.), and PEG 20 glyceryl monotallowate (commercially available as Varonic LI-42 from Sherex Chemical Company, Inc.).

The inventors have also discovered that a high molecular weight polyethylene glycol with an HLB value greater than 10, such as PEG 3400, may be employed as a surfactant to increase dispersibility of a polymer in an aqueous gel.

Polymerization of the monomers to prepare the crosslinked carboxylic copolymers used in the present invention is preferably carried out in a solvent in the presence of a free radical catalyst in an inert atmosphere in a closed reaction vessel equipped with a stirrer under autogenous pressure, artificially induced pressure, or in an open vessel equipped with a stirrer under an inert atmosphere or under reflux at atmospheric pressure. The polymerization temperature is preferably between from about 0°C to about 150°C, more preferably from about 25°C to about 100°C. The reaction can be conducted either batchwise or with the continuous addition of reactants.

Preferred radical catalysts for initiating the polymerization reaction include peroxide and hydroperoxide compounds such as benzoyl peroxide, lauroyl peroxide, caprylyl peroxide, tert-butyl peroxide, tert-butyl hydroperoxide; perbenzoates such as t-butyl perbenzoate; peroxyesters such as 2,5-dimethyl-2,5-bis(2-ethyl

hexoylperoxy)hexane; and azo compounds such as azobisisobutyronitrile and azobisisovaleronitrile. The initiators may be employed separately or in a mixture. The free radical generating initiator is preferably employed in an amount of from about 0.2% to about 1.2% by weight of the total monomers, more preferably from about 0.25% to about 0.75% by weight of the total monomer content, most preferably from about 0.3% to about 0.6% by weight of the total monomer content.

The polymerization reaction may be carried out in an organic solvent in which the monomers are soluble, but in which the polymers of this invention are insoluble. Consequently, the polymer precipitates out of the polymerization solvent in the form of fine particles. The solvent is then removed via filtration methods and the polymer is dried. The solvent employed must be inert to the monomers, the polymers, and the ethoxylated surfactant. Exemplary suitable solvents include aliphatic, cycloaliphatic, aromatic, halogen-substituted hydrocarbons, and alkyl-aromatic solvents such as hexane, heptane, octane, cyclohexane, benzene, toluene, xylene, chlorobenzene, carbon tetrachloride, trichloroethylene, and trichloroethane. Preferably, hexane, heptane, or cyclohexane is employed. Most preferably, cyclohexane is employed. The concentration of the monomers in the polymerization solvent is preferably from about 5% to 25%, more preferably from about 10% to about 25% by weight. The inventors have found that at higher concentrations, the polymerization reaction is strongly exothermic and too viscous.

If an ethoxylated surfactant is employed, it must be insoluble (incompatible) with the polymerization solvent. Preferably, the ethoxylated surfactant has an HLB value greater than 10, more preferably, from about 11 to about 20.

The crosslinked carboxylic polymers used in the invention enhance the appearance and performance of gel, cream and lotion formulations through improved thickening, improved emulsion stability, and elegant feel. When the crosslinked carboxylic copolymers are added to an aqueous system, thickening is obtained at a pH of from about 2 to about 3, but is greatly increased when the carboxylic acid groups in the polymer are neutralized, the optimum pH being between about 7 and about 9. Exemplary suitable neutralizing agents for use in the invention include aqueous ammonia, sodium hydroxide, potassium hydroxide, sodium carbonate, and amines such as monoethanolamine, diethanolamine, triethanolamine, monoethylamine, and the like. Through the use of primary, secondary, or tertiary organic amines, it is possible to thicken hydroalcoholic media with the crosslinked carboxyl polymers of the present invention.

The crosslinked carboxylic copolymers used in the invention are particularly effective in enhancing the appearance and performance of cream and lotion formulations through improved thickening efficiency, improved emulsion stability and elegant feel. Furthermore, the inventive lotion and cream formulations thickened with the crosslinked carboxyl copolymers exhibit excellent emulsion stability at both room temperature and elevated temperatures (40° and 50°C). The crosslinked carboxylic copolymers used in the present invention are effective in thickening aqueous media from about 0.1% by weight of the total weight of the medium to be thickened. The copolymers invention are generally employed in an amount of from about 0.1% to about 1.0% by weight of the total weight of the medium to be thickened.

The invention will be further clarified by the following examples, which are intended to be purely exemplary of the invention.

Example 1

500 ml of cyclohexane were charged into a 1-liter resin kettle equipped with a thermometer, condenser, two addition funnels, nitrogen inlet tube and mechanical stirrer. The solvent was heated to reflux (80°-81°C) under nitrogen. 46 g acrylic acid, 3.5 g vinyl acetate, 0.5 g triallylisocyanurate and 0.25 g Varonic LI-63 (PEG 30 glyceryl monococoate commercially available from Sherex Chemical Company, Inc.) were combined in a separate vessel and stirred until a homogeneous mixture was obtained. The homogeneous monomer mixture was placed in an addition funnel. 0.25 g of benzoyl peroxide was added to 10 ml cyclohexane and heated slowly with stirring to 70°C until dissolved. The heated initiator solution was transferred to a heated addition funnel. The heated initiator solution and the monomer mixture were added simultaneously over a period of 30 minutes to the reactor containing cyclohexane under reflux. The reaction mixture was then maintained at reflux (80°-81°C) for 4 hours and then allowed to cool. The white precipitate which formed during the reaction was filtered, washed with 50 ml of cyclohexane, and the resulting wet polymer cake was dried under vacuum at 80°C for 16 hours.

Samples of the polymer were added to deionized water at concentrations of 0.2% and 0.5% by weight. The solution was stirred at 600 rpm for 30 minutes to disperse the polymer completely. The polymer solutions were then neutralized with triethanolamine to a pH of 7. The solutions were stirred for an additional 20 minutes. The viscosity of this solution was measured with a Brookfield RVT viscometer at 20 rpm equipped with a #6 spindle in accordance with ASTM D 2196-81.

The viscosities of the gels prepared at 0.2% and 0.5% polymer concentrations are set forth in Table 1.

Example 2

500 ml of cyclohexane were charged into a 1-liter resin kettle equipped with a thermometer, condenser, two addition funnels, nitrogen inlet tube and mechanical stirrer. The solvent was heated to reflux (80-8.1°C) under nitrogen. 46 g acrylic acid, 3.5 g vinyl acetate, and 0.5 g triallylisocyanurate were combined in a separate vessel and stirred until a homogeneous mixture was obtained. This homogeneous monomer mixture was placed in an addition funnel. 0.25 g of benzoyl peroxide was added to 10 ml cyclohexane and heated slowly with stirring to 70°C until dissolved. This heated initiator solution and the monomer mixture were added simultaneously over a period of 30 minutes to the reactor containing cyclohexane under reflux. The reaction mixture was then maintained at reflux for 4 hours and then allowed to cool. The white precipitate which formed during the reaction was filtered, washed with 50 ml of cyclohexane and the resulting wet polymer cake was dried under vacuum at 80°C for 16 hours. Samples of the polymer were prepared as 0.2% and 0.5% polymer gels and evaluated in accordance with the procedures set forth in Example 1. The results are set forth in Table 1.

Example 3

3775 ml of cyclohexane were charged into a 5-liter resin kettle equipped with a thermometer, condenser, two addition funnels, nitrogen inlet tube and mechanical stirrer. The cyclohexane was heated to reflux (80-81°C) under nitrogen. 20 g 1-vinyl-2-pyrrolidinone, 5.3 g triallyl trimellitate, 374.7 g acrylic acid and 4 g PEG 3400 were combined in a separate vessel and stirred until a homogeneous mixture was obtained. 160 g (40% by weight) of the mixture were charged into the reaction kettle containing the cyclohexane. The remaining monomer mixture was placed in an addition funnel. 4 g of benzoyl peroxide were added to 225 ml cyclohexane and heated slowly to 70°C with stirring until dissolved. The heated initiator solution was . transferred to a second heated addition funnel. The heated initiator solution and the monomer mixture were added simultaneously over a period of 45 minutes to the stirred reaction mixture under reflux. The reaction was allowed to reflux with stirring for a total of 3 hours, which includes the time for the monomer addition. After 3 hours, the reaction mixture was allowed to cool to room temperature. The white precipitate which formed during the reaction was filtered through a Buchner funnel and dried under vacuum at room temperature for 16 hours. Samples of the polymer were prepared as 0.2% and 0.5% polymer gels and evaluated in accordance with the procedures set forth in Example 1. The results are set forth in Table 1.

Example 4

500 ml of cyclohexane were charged into a 1-liter resin kettle equipped with a thermometer, condenser, addition funnel, nitrogen inlet tube and a mechanical stirrer. The cyclohexane was heated to reflux (80°-81°C) under nitrogen. 49.51 g acrylic acid and 0.5 g triallyl isocyanurate were mixed in a separate vessel. 20 g (40% by weight) of the mixture were charged into the reaction kettle containing the cyclohexane under reflux. The remaining monomer mixture was placed in an addition funnel. 0.5 g of benzoyl peroxide was dissolved in 5 ml methyl ethyl ketone (to aid in the solubilizing of the initiator) and 5 ml cyclohexane. This initiator solution was placed in a syringe pump. The monomer mixture and the initiator solution were added over a period of 30 minutes to the stirred reaction mixture. The reaction was allowed to reflux for a total of 3 hours which includes the time for the monomer addition. At the end of 3 hours, the reaction mixture was allowed to cool to room temperature. The white precipitate, which formed during the reaction, was filtered through a Buchner funnel, washed with 50 ml cyclohexane, and dried under vacuum at room temperature for 16 hours. Samples of the polymer were prepared as 0.2% and 0.5% polymer gels and evaluated in accordance with the procedures set forth in Example 1. The results are set forth in Table 1.

Example 5

500 ml of cyclohexane were charged into a 1-liter resin kettle equipped with a thermometer, condenser, addition funnel, nitrogen inlet tube and mechanical stirrer. The cyclohexane was heated to reflux (80°-81°C) under nitrogen. 47.5 g acrylic acid, 2.5 g 1-vinyl-2-pyrrolidinone and 0.5 g triallyl isocyanurate were mixed in a separate vessel. 20 g (40% by weight) of the mixture were charged into the reaction kettle containing refluxing cyclohexane. The remaining monomer mixture was placed in an addition funnel. 0.5 g of benzoyl peroxide were dissolved in 5 ml methyl ethyl ketone (to aid in solubilizing the initiator in cyclohexane) and 5 ml cyclohexane. The initiator solution was placed in a syringe pump. The monomer mixture and the initiator solution were added

simultaneously over a period of 30 minutes to the stirred reaction mixture. The reaction was allowed to reflux for a total of 3 hours which includes the monomer additions. At the end of 3 hours, the reactor contents were allowed to cool to room temperature. The white precipitate which formed during the reaction was filtered through a Buchner funnel and dried under vacuum at room temperature for 16 hours. Samples of the polymer were prepared as 0.2% and 0.5% polymer gels and evaluated in accordance with the procedures set forth in Example 1. The results are set forth in Table 1.

Example 6

500 ml of cyclohexane were charged into a 1-liter resin kettle equipped with a thermometer, condenser, two addition funnels, nitrogen inlet tube and mechanical stirrer. The cyclohexane was heated to 70°C under nitrogen. 0.5 g of glyoxal bis(diallyl acetal) and 3.5 g of vinyl acetate were added to the kettle. 0.25 g of benzoyl peroxide were dissolved in 2.5 ml methyl ethyl ketone and 2.5 ml cyclohexane. The mixture was added to the reactor over a period of 15 minutes via a syringe pump. After addition was completed, the reaction mixture was slowly heated to reflux (80°-81°C). 46 g of acrylic acid were placed in an additional funnel and another portion of the initiator solution (0.25 g of benzoyl peroxide dissolved in 2.5 ml methyl ethyl ketone and 2.5 ml cyclohexane) was placed in a syringe pump. The two materials were added simultaneously over a period of 30 minutes and the reaction was allowed to proceed for 4 hours at reflux and then the reactor contents were allowed to cool to room temperature. The white precipitate which formed during the reaction was filtered through a Buchner funnel and dried under vacuum at room temperature for 16 hours. Samples of the polymer were prepared as 0.2% and 0.5% polymer gels and evaluated in accordance with the procedures set forth in Example 1. The results are set forth in Table 1.

Comparative Example A

500 ml of cyclohexane were charged into a 1-liter resin kettle equipped with a thermometer, condenser, addition funnel, nitrogen inlet tube and mechanical stirrer. The cyclohexane was heated to 70°C under nitrogen. 0.5 g of triallyl cyanurate and 2 g of vinyl acetate were mixed and the monomer mixture was charged to the kettle. 0.25 g of benzoyl peroxide were dissolved in 2.5 ml methyl ethyl ketone and 2.5 ml cyclohexane mixture were added to the reactor over a period of 15 minutes via a syringe pump. After the addition was completed, the reaction mixture was slowly heated to reflux (80°-81°C). 47.5 g of acrylic acid and 0.5 g of acrylamide were homogeneously mixed and the monomer mixture was transferred to an addition funnel. A second portion of the initiator solution (0.25 a of benzoyl peroxide dissolved in 2.5 ml methyl ethyl ketone and 2.5 ml cyclohexane) was placed in a syringe pump. The monomer solution and the initiator solution were simultaneously added to the reaction mixture over a period of 30 minutes. After the additions were completed, the reaction was allowed to proceed for 4 hours at reflux and then the reactor contents were allowed to cool to room temperature. The white precipitate which formed during the reaction was filtered through a Buchner funnel and dried under vacuum at room temperature for 16 hours. Samples of the polymer were prepared as 0.2% and 0.5% polymer gels and evaluated in accordance with the procedures set forth in Example 1. The results are set forth in Table 1.

Comparative Example B

500 ml of cyclohexane were charged into a 1-liter resin kettle equipped with a thermometer, condenser, nitrogen inlet tube and mechanical stirrer. The cyclohexane was heated to reflux (80°-81°C) under nitrogen. 46 g acrylic acid, 3.5 g vinyl acetate and 0.5 g isoprene were mixed homogeneously in a separate vessel and placed in a syringe pump. 0.25 g of benzoyl peroxide was dissolved in 2.5 ml methyl ethyl ketone and diluted with 2.5 ml cyclohexane. The initiator solution was placed in a second syringe pump. The initiator solution and the monomer mixture were added simultaneously over a period of 30 minutes to the reactor which contained cyclohexane at reflux temperature. At the completion of the addition, the reaction was allowed to proceed for 4 hours at reflux and then the contents were allowed to cool to room temperature. The white precipitate which formed during the reaction was filtered through a Buchner funnel and dried under vacuum at 50°C for 16 hours. 0.2% and 0.5% polymer gels were prepared in accordance with the procedures set forth in Example 1. The results are set forth in Table 1.

Comparative Example C

500 ml of cyclohexane were charged into a 1-liter resin kettle equipped with a thermometer, condenser,

nitrogen inlet tube and mechanical stirrer. The cyclohexane was heated to reflux (80°-81°C) under nitrogen. 46 g acrylic acid, 3.5 g vinyl acetate and 0.5 g divinyl benzene were homogeneously mixed together and placed in a syringe pump fitted with a 50 ml syringe. 0.25 g of benzoyl peroxide were dissolved in 2.5 ml methyl ethyl ketone and diluted with 2.5 ml cyclohexane. The initiator solution was placed in a syringe pump fitted with a 5 ml syringe. The initiator solution and the monomer mixture were added simultaneously over a period of 30 minutes to the reactor which contained cyclohexane at reflux temperature. After the addition was completed, the reaction was allowed to proceed for 4 hours at reflux and then the contents were allowed to cool to room temperature. The white precipitate which formed during the reaction was filtered through a Buchner funnel and dried under vacuum at 50°C for 16 hours. 0.2% and 0.5% polymer gels were prepared and evaluated in accordance with the procedures set forth in Example 1. The results are set forth in Table 1.

Comparative Example D

0.2% and 0.5% polymer gels of Carbopol 934 (a lightly crosslinked acrylic polymer commercially available from B.F. Goodrich, Inc.) were prepared and evaluated in accordance with the procedures set forth in Example 1. The results are set forth in Table 1.

TABLE 1

THICKENING EFFICIENCY IN AQUEOUS GELS NEUTRALIZED WITH TRIETHANOLAMINE

| Example | Brookfield Viscosity, 20 rpm (mPa.s,cP) 0.2% Concentration | 0.5% Concentration |
|---|---|---|
| 1 | 9,000 | 34,500 |
| 2 | 11,500 | 32,000 |
| 3 | 3,000 | 30,250 |
| 4 | | 48,000 |
| 5 | | 37,500 |
| 6 | | 36,500 |
| Comp.A | 4,500 | 28,000 |
| Comp.B | | 75 |
| Comp.C | | 350 |
| Comp.D | 2,300 | 28,250 |

Example 7

The carboxylic polymer prepared in accordance with Example 1 was incorporated into Formulation A, an emollient lotion formulation, at concentrations of 0.05, 0.10, 0.15 and 0.20% by weight of the total formulation, and evaluated for thickening efficiency and heat stability at 50°C. A failure in the emulsion stability occurred when separation or syneresis occurred.

## Formulation A: Emollient Lotion

| Water Phase | % |
|---|---|
| 1. Deionized Water* | 87.5 |
| 2. Glycerine | 3.0 |
| 3. Triethanolamine | 1.2 |
| 4. Methyl Paraben | 0.1 |
| 5. Carboxylic Polymer | 0.05, 0.10, 0.15, 0.20 |

| Oil Phase | |
|---|---|
| 1. Mineral Oil | 4.0 |
| 2. Stearic Acid (Triple XXX) | 1.5 |
| 3. Glyceryl Monostearate | 1.5 |
| 4. Cetyl Alcohol | 0.7 |
| 5. Dow Corning Fluid 200 (1000 cs) | 0.3 |
| 6. Propyl Paraben | 0.1 |

Procedure:

1. The carboxylic polymer was dispersed in water/glycerine at 600 rpm and heated to 75-80°C. Methyl paraben was then added.
2. The oil phase was heated to 80-85°C.
3. The oil phase was added to the water phase with rapid stirring at 750 rpm.

* The amount of water was adjusted for the various polymer loading levels so that the total weight was 100%.

4. The mixture was cooled to 55-65°C and triethanolamine was added.
5. With continued stirring, the lotion was cooled to 35-40°C.
6. The lotion was allowed to stabilize at room temperature overnight.
7. The samples to be tested for heat stability were placed in an oven at 50°C.

The Brookfield viscosities at 20 rpm of the lotion are set forth in Table 2. The results of the heat stability

at 50°C of the formulations are set forth in Table 3.

Example 8

The carboxylic polymer prepared in accordance with Example 2 was incorporated into Formulation A at concentrations of 0.05, 0.10, 0.15 and 0.20% by weight and evaluated for thickening efficiency and heat stability at 50°C.

The Brookfield viscosities at 20 rpm of the lotions are set forth in Table 2. The results of the heat stability at 50°C of the formulations are set forth in Table 3.

Comparative Example E

The carboxylic polymer of Comparative Example D was incorporated into Formulation A at concentrations of 0.05, 0.10, 0.15 and 0.20% and evaluated for thickening efficiency and heat stability at 50°C.

The Brookfield viscosities at 20 rpm of the lotions are set forth in Table 2. The results of the heat stability at 50°C of the formulations are set forth in Table 3.

TABLE 2

THICKENING EFFICIENCY IN EMOLLIENT LOTION FORMULATION A

| Example | Carboxylic Polymer | Brookfield Viscosity, 20 rpm (mPa.s.cP) | | | |
|---|---|---|---|---|---|
| | | 0.05% | 0.1% | 0.15% | 0.2% (Polymer Conc.) |
| 7 | Example 1 | 4,600 | 7,200 | 10,800 | 14,000 |
| 8 | Example 2 | 4,000 | 7,800 | 10,200 | 13,000 |
| Comp.E | Comp. Ex.D | 5,000 | 5,800 | 9,400 | 14,800 |

TABLE 3

HEAT STABILITY OF EMOLLIENT LOTION FORMULATION A

| Example | Carboxylic Polymer | Heat Stability (50°C), Days | | |
|---|---|---|---|---|
| | | 0.1% | 0.15% | 0.2% (Polymer Conc.) |
| 7 | Example 1 | 30 | 150 | 235 |
| Comp.E | Comp. Ex.D | 12 | 16 | 20 |

Example 9

The carboxylic polymer prepared in accordance with Example 1 was incorporated into Formulation B, a moisturizing cream formulation, at concentrations of 0.05, 0.10, 0.15 and 0.20% by weight of the total formulation and evaluated for thickening efficiency and heat stability at 50°C.

The Brookfield viscosities at 20 rpm of the creams are set forth in Table 4. The results of the heat stabilities at 50°C of the formulations are set forth in Table 5.

FORMULATION B: MOISTURIZING CREAM FORMULATION

| Water Phase | % |
|---|---|
| 1. Deionized Water** | 67.2 |
| 2. Glycerine | 11.5 |
| 3. Triethanolamine | 2.4 |
| 4. Carboxylic Polymer | 0.05, 0.1, 0.15, 0.2 |
| 5. Methyl Paraben | 0.2 |
| **Oil Phase** | |
| 1. Amerchol L-101*** | 10.5 |
| 2. Cetyl Alcohol | 5.0 |
| 3. Stearic Acid (Triple XXX) | 3.0 |
| 4. Propyl Paraben | 0.1 |

Procedure:

1. The carboxylic polymer was dispersed in water/glycerine at 600 rpm and heated to 75-80°C. Methyl paraben was added.
2. The oil phase was heated to 80-85°C.
3. The oil phase was added to the water phase with rapid stirring at 750 rpm.

---

** The amount of water was adjusted for the various polymer loading levels so that the total weight was 100%.

*** Mineral Oil and Lanolin Alcohol.

4. The mixture was cooled to 55-65°C and triethanolamine was added.
5. With continued stirring, the lotion was cooled to 40°C.
6. The lotion was allowed to stabilize at room temperature overnight.
7. The samples to be tested for heat stability were placed in an oven at 50°C.

Example 10

The carboxylic polymer prepared in accordance with Example 2 was incorporated into Formulation B, a moisturizing cream formulation, at concentrations of 0.05, 0.10, 0.15 and 0.20% by weight of the total formulation and evaluated for thickening efficiency and heat stability at 50°C.

The Brookfield viscosities at 20 rpm of the creams are presented in Table 4. The results of the heat stabilities at 50°C of the formulation are presented in Table 5.

Comparative Example F

Carbopol 941 (a lightly crosslinked acrylic polymer commercially available from B.F. Goodrich, Inc.) was incorporated into Formulation B, a moisturizing cream formulation, at concentrations of 0.05, 0.10, 0.15 and 0.20% by weight of the total formulation and evaluated for thickening efficiency and heat stability at 50°C.

The Brookfield viscosities at 20 rpm of the creams are set forth in Table 4. The heat stability at 50°C results are set forth in Table 5.

TABLE 4

THICKENING EFFICIENCY IN MOISTURIZING CREAM FORMULATION B

| Example | Carboxylic Polymer | Brookfield viscosity, 20 rpm mPa.s,cP) | | | |
|---|---|---|---|---|---|
| | | 0.05% | 0.1% | 0.15% | 0.2% (Polymer Conc.) |
| 9 | Example 1 | 116,000 | 208,000 | 304,000 | 376,000 |
| 10 | Example 2 | 110,000 | 234,000 | 325,000 | 400,000 |
| Comp.I | Comp. Ex.I | 92,000 | 130,000 | 144,000 | 172,000 |

TABLE 5

HEAT STABILITY OF MOISTURIZING CREAM FORMULATION B

| Example | Carboxylic Polymer | Heat Stability (50°C), Days | | |
|---|---|---|---|---|
| | | 0.1% | 0.15% | 0.2% (Polymer Conc.) |
| 9 | Example 1 | 46 | 56 | 70 |
| Comp.I | Comp. Ex.I | 24 | 28 | 35 |

Other embodiments of the invention will be apparent to those skilled in the art from consideration of the specification and practice of the invention disclosed herein. It is intended that the specification and examples be considered as exemplary only, with the true scope and spirit of the invention being indicated by the following claims.

## Claims

1. A cosmetic or pharmaceutical formulation for application to the skin, said formulation being in the form of a thickened emulsion comprising a crosslinked carboxylic copolymer formed from a mixture of monomers comprising
   (a) a mono olefinically unsaturated carboxylic acid;
   (b) at least one crosslinking monomer containing at least two ethylenically unsaturated groups;
   (c) a comonomer containing a polymerizable ethylenically unsaturated group selected from the group consisting of vinylacetate, vinylpyrrolidone, methyl vinyl ether, ethyl vinyl ether, acrylamide, methacrylamide and methyl vinyl ketone;

   characterized in that
   the crosslinking monomer is selected from the group consisting of triallylisocyanurate, triallyl trimellitate and glyoxal bis(diallyl acetal) and is present in an amount sufficient to thicken said formulation and the mixture optionally further comprises an ethoxylated glyceride compound.

2. Formulation according to claim 1, characterized in that said crosslinking monomer is triallylisocyanurate.

3. Formulation according to claim 1 or 2, characterized in that said olefinically unsaturated acid is selected

from the group consisting of acrylic acid, methacrylic acid, ethacrylic acid, fumaric acid, crotonic acid, itaconic acid, styrylacrylic acid, glutaconic acid, maleic acid and maleic anhydride.

4. Formulation according to any one of claims 1 to 3, characterized in that said unsaturated carboxylic acid is present in an amount of from about 70 % to about 99 % by weight, and said crosslinking monomer is present in an amount of from about 0.8 % to about 1.2 % by weight.

5. Formulation according to claim 4, characterized in that said unsaturated carboxylic acid is present in an amount of from about 92 % to about 95 % by weight, and said crosslinking monomer is present in an amount of from about 0.9 % to about 1.1 % by weight.

6. Formulation according to any one of claims 1 to 5, characterized in that the comonomer containing a polymerizable ethylenically unsaturated group is present in an amount of from 0.1 % to about 29 % by weight.

7. Formulation according to claim 6, characterized in that the comonomer containing a polymerizable ethylenically unsaturated group is present in an amount of from 2 % to about 15 % by weight.

8. Formulation according any one of claims 1 to 7, characterized in that said ethoxylated glyceride compound is of the formula (I):

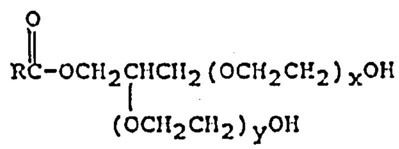

(I)

wherein R represents an alkyl group of from $C_8$ to $C_{18}$ and the sum of x + y is from 20 to 300.

9. Formulation according any one of claims 1 to 8, characterized in that said ethoxylated glyceride compound is present in an amount of 0.1 % to about 1.0% by weight of the formulation.

10. Formulation according claim 9, characterized in that said ethoxylated glyceride compound is present in an amount of 0.5 % to about 0,75 % by weight of the formulation.

11. Formulation according any one of claims 1 to 10 having a pH of from about 7 to about 9.

**Patentansprüche**

1. Kosmetische oder pharmazeutische, zur Anwendung auf der Haut bestimmte Zubereitung in der Form einer verdickten Emulsion, welche ein vernetztes carboxylisches Copolymerisat enthält, das aus einem aus
   (a) einer monoolefinisch ungesättigten Carbonsäure;
   (b) mindestens einem mindestens zwei ethylenisch ungesättigte Gruppen enthaltenden vernetzenden Monomer;
   (c) einem eine polymerisierbare, ethylenisch ungesättigte, aus der Reihe Vinylacetat, Vinylpyrrolidon, Methylvinylether, Ethylvinylether, Acrylamid, Methacrylamid und Methylvinylketon gewählte Gruppe enthaltenden Comonomer;

   bestehenden Monomergemisch gebildet wird,
   dadurch gekennzeichnet, daß
   das vernetzende Monomer aus der Reihe Isocyanursäuretriallylester, Trimellitsäuretriallylester und Glyoxal-bis (diallylacetal) gewählt wird und in einer zur Verdickung der genannten Zubereitung ausreichenden Menge vorliegt und
   das Gemisch gegebenenfalls zusätzlich eine ethoxylierte Glyceridverbindung enthält.

2. Zubereitung nach Anspruch 1, dadurch gekennzeichnet, daß das vernetzende Monomer Isocyanursäuretriallylester ist.

3. Zubereitung nach Anspruch 1 oder 2, dadurch gekennzeichnet, daß die olefinisch ungesättigte Säure aus der Reihe Acrylsäure, Methacrylsäure, Ethacrylsäure, Fumarsäure, Krotonsäure, Itakonsäure,

Styrylacrylsäure, Glutakonsäure, Maleinsäure und Maleinsäureanhydrid gewählt wird.

4. Zubereitung nach einem der Ansprüche 1 bis 3, dadurch gekennzeichnet, daß die ungesättigte Carbonsäure in einer Menge zwischen etwa 70 Gew.-% und etwa 99 Gew.-% vorliegt, und das vernetzende Monomer in einer Menge zwischen etwa 0,8 Gew.-% und etwa 1,2 Gew.-% vorliegt.

5. Zubereitung nach Anspruch 4, dadurch gekennzeichnet, daß die ungesättigte Carbonsäure in einer Menge zwischen etwa 92 Gew.-% und etwa 95 Gew.-% vorliegt, und das vernetzende Monomer in einer Menge zwischen etwa 0,9 Gew.-% und etwa 1,1 Gew.-% vorliegt.

6. Zubereitung nach einem der Ansprüche 1 bis 5, dadurch gekennzeichnet, daß das eine polymerisierbare, ethylenisch ungesättigte Gruppe enthaltende Comonomer in einer Menge zwischen 0,1 Gew.-% und etwa 29 Gew.-% vorliegt.

7. Zubereitung nach Anspruch 6, dadurch gekennzeichnet, daß das eine polymerisierbare ethylenisch ungesättigte Gruppe enthaltende Comonomer in einer Menge zwischen 2 Gew.-% und etwa 15 Gew.-% vorliegt.

8. Zubereitung nach einem der Ansprüche 1 bis 7, dadurch gekennzeichnet, daß die ethoxylierte Glyceridverbindung die Formel (I) aufweist:

$$\overset{\displaystyle O}{\overset{\|}{RC}}-OCH_2\underset{\displaystyle (OCH_2CH_2)_yOH}{\underset{|}{C}}HCH_2(OCH_2CH_2)_xOH \qquad (I)$$

wobei R für eine Alkylgruppe zwischen $C_8$ und $C_{18}$ steht und die Summe x + y zwischen 20 und 300 liegt.

9. Zubereitung nach einem der Ansprüche 1 bis 8, dadurch gekennzeichnet, daß die ethoxylierte Glyceridverbindung in einer Menge von 0,1 Gew.-% bis etwa 1,0 Gew.-% der Zubereitung vorliegt.

10. Zubereitung nach Anspruch 9, dadurch gekennzeichnet, daß die ethoxylierte Glyceridverbindung in einer Menge von 0,5 Gew.-% bis etwa 0,75 Gew.-% der Zubereitung vorliegt.

11. Zubereitung nach einem der Ansprüche 1 bis 10 mit einem pH-Wert zwischen etwa 7 und etwa 9.

## Revendications

1. Formulation cosmétique ou pharmaceutique pour application à la peau, ladite formulation étant sous la forme d'une émulsion épaissie comprenant un copolymère carboxylique réticulé formé à partir d'un mélange de monomères comprenant:
   (a) un acide carboxylique mono-oléfiniquement insaturé;
   (b) au moins un monomère réticulant contenant au moins deux groupes éthyléniquement insaturés;
   (c) un comonomère contenant un groupe éthyléniquement insaturé polymérisable choisi dans le groupe constitué par l'acétate de vinyle, la vinylpyrrolidone, l'éther méthyl-vinylique, l'éther éthyl-vinylique, l'acrylamide, le méthacrylamide et la méthyl-vinyl-cétone;
   caractérisée en ce que
   le monomère réticulant est choisi dans le groupe constitué par l'isocyanurate de triallyle, le trimellitate de triallyle et le glyoxal-bis(diallylacétal) et est présent en une quantité suffisante pour épaissir ladite formulation et
   le mélange comprend en outre facultativement un composé de glycéride éthoxylé.

2. Formulation selon la revendication 1, caractérisée en ce que ledit monomère réticulant est l'isocyanurate d'isoallyle.

3. Formulation selon la revendication 1 ou 2, caractérisée en ce que ledit acide oléfiniquement insaturé est choisi dans le groupe constitué par l'acide acrylique, l'acide méthacrylique, l'acide éthacrylique, l'acide

fumarique, l'acide crotonique l'acide itaconique, l'acide styrylacrylique, l'acide glutaconique, l'acide maléique et l'anhydride maléique.

**4.** Formulation selon l'une quelconque des revendications 1 à 3, caractérisée en ce que ledit acide carboxylique insaturé est présent en une quantité d'environ 70% à environ 99% en poids, et en ce que ledit monomère réticulant est présent en une quantité d'environ 0,8% à environ 1,2% en poids.

**5.** Formulation selon la revendication 4, caractérisée en ce que ledit acide carboxylique insaturé est présent en une quantité d'environ 92% à environ 95% en poids, et en ce que ledit monomère réticulant est présent en une quantité d'environ 0,9% à environ 1,1% en poids.

**6.** Formulation selon l'une quelconque des revendications 1 à 5, caractérisée en ce que le comonomère contenant un groupe éthyléniquement insaturé polymérisable est présent en une quantité de 0,1% à environ 29% en poids.

**7.** Formulation selon la revendication 6, caractérisée en ce que le comonomère contenant un groupe éthyléniquement insaturé polymérisable est présent en une quantité allant de 2% à environ 15% en poids.

**8.** Formulation selon l'une quelconque des revendications 1 à 7, caractérisée en ce que ledit composé de glycéride éthoxylé est de formule (I):

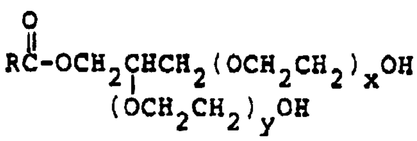

(I)

dans laquelle R représente un groupe alcoyle en $C_8$ à $C_{18}$ et la somme de x + y vaut de 20 à 300.

**9.** Formulation selon l'une quelconque des revendications 1 à 8, caractérisée en ce que ledit composé de glycéride éthoxylé est présent en une quantité de 0,1% à environ 1,0% en poids de la formulation.

**10.** Formulation selon la revendication 9, caractérisée en ce que ledit composé de glycéride éthoxylé est présent en une quantité de 0,5% à environ 0,75% en poids de la formulation.

**11.** Formulation selon l'une quelconque des revendications 1 à 10 ayant un pH d'environ 7 à environ 9.